# EUROPEAN PATENT SPECIFICATION

(11) **EP 4 233 844 B1**
(45) Date of publication and mention of the grant of the patent: **12.03.2025**
(21) Application number: 20960660.7
(22) Date of filing: 10.12.2020
(51) Int. Cl.: A61K 9/16, A61K 31/519, A61K 31/485, A61K 47/34, A61K 47/32, A61P 25/36, A61K 9/20, A61P 25/30, A61K 9/50, A61K 9/28

(54) **NALTREXONE AND RISPERIDONE COMBINATION SUSTAINED-RELEASE COMPOSITION**
ZUSAMMENSETZUNG MIT VERZÖGERTER FREISETZUNG AUS EINER NALTREXON- UND RISPERIDONKOMBINATION
COMPOSITION À LIBÉRATION PROLONGÉE DE COMBINAISON DE NALTREXONE ET DE RISPÉRIDONE

(30) Priority: 09.11.2020 CN 202011243436
(43) Date of publication of application: 30.08.2023
(73) Proprietor: Shenzhen Sciencare Pharmaceutical Co., Ltd., Shenzhen Guangdong 518000 (CN)
(72) Inventor: YAN, Xieguo, Shenzhen, Guangdong 518000 (CN); YAN, Fuqiao, Shenzhen, Guangdong 518000 (CN); LIU, Guohui, Shenzhen, Guangdong 518000 (CN); WANG, Shiqiang, Shenzhen, Guangdong 518000 (CN)
(74) Representative: karo IP
(86) International application number: PCT/CN2020/135369
(87) International publication number: WO 2022/095203

(56) References cited:
- WO-A1-2019/231993
- CN-A- 101 292 960
- CN-A- 101 420 944
- CN-A- 101 420 944
- CN-A- 101 700 226
- CN-A- 102 512 399
- CN-A- 102 512 399
- CN-A- 106 474 070
- CN-A- 106 474 070
- CN-A- 110 123 789
- CN-A- 110 742 871
- CN-A- 111 329 845
- CN-A- 112 245 434
- US-A1- 2003 211 164
- US-A1- 2010 076 006

## Description

### Invention Field

The present disclosure relates to a compound sustained-release composition of naltrexone and risperidone prepared by a method, according to claim 1, and to its medical use, according to claim 10.

### Background

Naltrexone is a pure antagonist for opioid receptors, and has a block effect on µ-, δ-, and x-opioid receptors. Naltrexone can block the effect of retaking drugs, thereby weakening the positive reinforcement and negative reinforcement, and playing a good supporting role in prevention of relapse. Naltrexone has the following chemical structure:

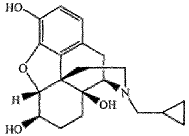

Risperidone has a good therapeutic effect on positive and negative symptoms and accompanying affective symptoms thereof (such as anxiety and depression). Risperidone may also reduce affective symptoms associated with schizophrenia. For patients who benefit from treatment during the acute period, this product can continue to exert its clinical efficacy during the maintenance period. Risperidone has the following chemical structure:

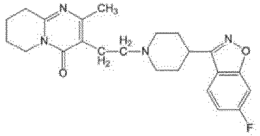

After intramuscular or subcutaneous injection, conventional injectable preparations, such as solution, suspension, and emulsion, will be removed from the body quickly. Therefore, frequently parenteral administration is required by treatment of chronic diseases. To solve the foregoing problem, sustained-release microparticles are proposed, which specifically refer to microcapsules prepared by encapsulating drugs into polymers or microsphere disperse systems prepared by dispersing or adsorbing drugs into polymers. Microspheres usually have a size in a unit of µm, so they can be administered to the human body or animals by intramuscular or subcutaneous injection. Microspheres may be prepared according to different drug release rates, so that the drug delivery time can be controlled. Therefore, the effective therapeutic concentration of a drug can be maintained for a long term after single administration, which reduces the total dose of the drug required by treatment to the maximum extent, and improves the patient compliance with drug therapy.

The approved VIVITROL^{®} includes the active ingredient naltrexone, and it is proved that the preparation has a good effect of improving opioid relapse control and quitting alcohol addiction. However, the preparation cannot achieve good results in treatment of novel drug addiction, and especially cannot achieve satisfactory results in treatment of methamphetamine drug addiction. US 2003/211164 A1 discloses microparticles comprising naltrexone or risperidone. Microparticles comprising naltrexone are further disclosed in CN 101 700 226 A, CN 102 512 399 A and CN 110 742 871 A. Microparticles comprising risperidone are further disclosed in CN 106 474 070 A and CN 110 123 789 A.

Currently, there is no report on application of a compound composition of naltrexone and risperidone in addiction treatment.

### Summary

A technical problem to be solved by the present disclosure is to provide a naltrexone sustained-release microsphere preparation capable of effectively prolonging the in vivo action time of a compound of naltrexone and risperidone to reduce the administration frequency of naltrexone and risperidone.

An objective of the present disclosure is to provide a compound sustained-release composition of naltrexone and risperidone, which includes the following components in parts by weight:
5-10 parts of naltrexone sustained-release microspheres, and
1-2 parts of risperidone sustained-release microspheres, wherein the risperidone sustained-release microspheres are prepared by a W₁/O/W₂ double emulsion-solvent evaporation method, and
wherein the naltrexone sustained-release microspheres are prepared by an O/W emulsion-solvent evaporation method.

Further, a preparation method of the risperidone sustained-release microspheres
may include the following steps:
preparation of the risperidone sustained-release microspheres by a W₁/O/W₂ double emulsion-solvent evaporation method.
S1: dissolving an additive in purified water to form an inner water phase;
S2: dissolving risperidone and a fat-soluble polymer in an organic solvent to form an oil phase;
S3: adding the inner water phase to the oil phase, and performing ultrasonic emulsification to obtain a primary emulsion; and
S4: adding dropwise the primary emulsion to an aqueous solution of a water-soluble polymer, stirring until uniform, evaporating the organic solvent, washing, collecting, and drying to obtain risperidone sustained-release microspheres.

Further, the additive may be selected from at least one of sodium chloride, mannitol,
disodium hydrogen phosphate, and sodium dihydrogen phosphate.

Further, the fat-soluble polymer may be selected from at least one of polylactic acid
(PLA), poly(lactic-co-glycolic acid) (PLGA), polycaprolactone (PCL), polycarbonate, polyglycolic acid (PGA), polycyanoacrylate, and polyether ester.

Further, the water-soluble polymer may be selected from polyvinyl alcohol.

Further, in the poly(lactic-co-glycolic acid) (PLGA), a ratio of lactic acid to glycolic acid may be (1-9): 1.

Further, the weight-average molecular weight (M_{w}) of the water-soluble polymer
or the fat-soluble polymer may be
6,000-220,000 Da, and preferably 50,000-100,000 Da.

Further, the organic solvent may be
selected from at least one of dichloromethane and
ethyl acetate.

Further, in S1, a dosage ratio of the additive to the purified water may be 50-300 mg/mL,
and preferably 75-150 mg/mL.

Further, in S2, the concentration of risperidone may be
100-300 mg/mL, and preferably
100-150 mg/mL; and the concentration of the fat-soluble polymer may be 100-300 mg/mL,
and preferably 100-150 mg/mL.

Further, in S4, a dosage ratio of the primary emulsion to the aqueous solution of the water-soluble polymer may be (1-3): 80, and preferably (2-3): 80.

Further, in S4, in the aqueous solution of the water-soluble polymer, the content of the water-soluble polymer may be 0.1-2 wt%.

Further, in S4, the content of the water-soluble polymer may be 0.5-0.6 wt%.

Further, the aqueous solution may contain 0-5 wt% sodium chloride or 0-10 wt%
sucrose.

Further, the stirring may be performed at 500-5,000 revolutions/minute, and preferably
700-1,000 revolutions/minute, and the evaporation may be performed at 200-3,000
revolutions/minute, preferably 500-1,000 revolutions/minute, at 30-40°C for 24 h.

Further, a preparation method of the naltrexone sustained-release microspheres may include the following steps:
preparation of naltrexone sustained-release microspheres by an O/W emulsion-solvent evaporation method.
S1': dissolving naltrexone and a fat-soluble polymer in an organic solvent to form an oil phase; and
S2': adding dropwise the oil phase to an aqueous solution of a water-soluble polymer, stirring until uniform, evaporating the organic solvent, washing, collecting, and drying to obtain naltrexone sustained-release microspheres.

Further, the fat-soluble polymer may be selected from at least one of polylactic acid (PLA), poly(lactic-co-glycolic acid) (PLGA), polycaprolactone (PCL), polycarbonate, polyglycolic acid (PGA), polycyanoacrylate, and polyether ester.

Further, the water-soluble polymer may be selected from polyvinyl alcohol.

Further, the organic solvent may be selected from at least one of dichloromethane and ethyl acetate.

Further, in the poly(lactic-co-glycolic acid) (PLGA), a ratio of lactic acid to glycolic acid may be (1-9): 1.

Further, the weight-average molecular weight (M_{w}) of the water-soluble polymer or the fat-soluble polymer may be 6,000-220,000 Da, and preferably 50,000-100,000 Da.

Further, in S1', the concentration of naltrexone may be 50-300 mg/mL, and preferably 100-150 mg/mL; and the concentration of the fat-soluble polymer may be 100-300 mg/mL, and preferably 100-150 mg/mL.

Further, in S2', a dosage ratio of the oil phase to the aqueous solution of the water-soluble polymer may be (1-3): 80, and preferably (2-3): 80.

Further, in S2', in the aqueous solution of the water-soluble polymer, the content of the water-soluble polymer may be 0.1-2 wt%, and preferably 0.5-1 wt%.

Further, the aqueous solution may contain 0-10 wt% sodium chloride or 0-20 wt% sucrose, preferably 8-10 wt% sucrose.

Further, the stirring may be performed at 500-5,000 revolutions/minute, and preferably 700-1,000 revolutions/minute, and the evaporation may be performed at 200-3,000 revolutions/minute, preferably 500-1,000 revolutions/minute, at 30-40°C for 24 h.

Another objective of the present disclosure is to provide a preparation including the composition of the invention, which is prepared by uniformly mixing the composition with a lubricant, compressing into tablets, and coating.

Further, the lubricant may be selected from at least one of sodium stearyl fumarate, magnesium stearate, and a stearic acid.

Further, a dosage of lubricant may be is 0.05-0.1 wt% of the composition.

Still another objective of the present disclosure is to provide a medical use of the composition of the invention for treating or relieving amphetamine, ketamine, cocaine or cannabis addiction.

The present disclosure has the following advantages.

Patients with opioid or drug addiction usually develop psychiatric symptoms in addition to addiction symptoms. Therefore, during treatment of patients with opioid or drug addiction, ancillary interventions for mental stability of the patients are also important. In the present disclosure, naltrexone and risperidone are combined into a compound for use, which further improve the therapeutic effect on patients with opioid or drug addiction.
1. The sustained-release preparation of the present disclosure is composed of naltrexone, risperidone, and preferably a biodegradable pharmaceutical polymer material, and preferably an additive.
2. The preparation method of the present disclosure improves the stability of naltrexone and risperidone in the preparation process and release process, is simple and easy to operate, and has good repeatability.
3. The sustained-release microspheres of the present disclosure may be sustainably released in vitro for more than 12 weeks, the release conforms to a zeroth-order approximation mode, and the release rate is stable.
4. The suitable preparation method and parameter control are adopted in the present disclosure, so that naltrexone sustained-release microspheres and risperidone sustained-release microspheres are released synchronously, and an administration period is easier to control to avoid an uncertain administration period due to non-synchronous release of the two drugs.
5. In view of the fact that there is no anti-amphetamine relapse product on the market, the preparation of the present disclosure can better help patients avoid relapse during the window period due to frequent medications by virtue of its sustained-release characteristics.

### Brief Description of the Drawings

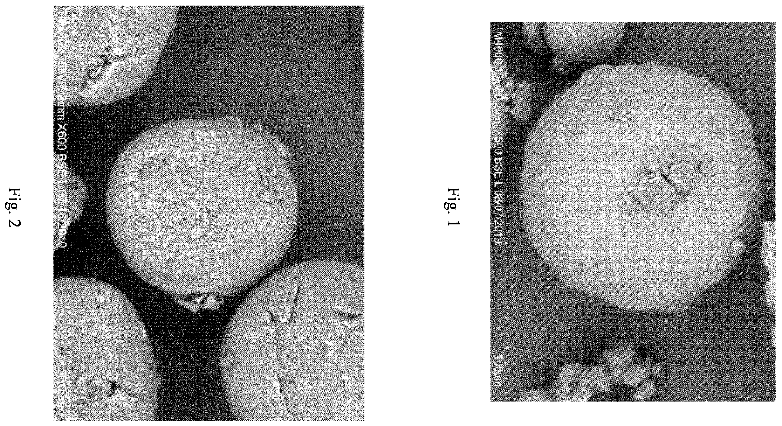

Fig. 1 shows a scanning electron microscopy image of naltrexone sustained-release microspheres prepared in Example 3 of the present disclosure;
Fig. 2 shows a scanning electron microscopy image of risperidone sustained-release microspheres prepared in Example 3 of the present disclosure;
Fig. 3 shows an in vitro cumulative release curve of a compound sustained-release preparation of naltrexone and risperidone prepared in Example 1 of the present disclosure, where the Y-axis represents a cumulative release percentage (%), and the X-axis represents time (day);
Fig. 4 shows an in vitro cumulative release curve of a compound sustained-release preparation of naltrexone and risperidone prepared in Example 2 of the present disclosure, where the Y-axis represents a cumulative release percentage (%), and the X-axis represents time (day);
Fig. 5 shows an in vitro cumulative release curve of a compound sustained-release preparation of naltrexone and risperidone prepared in Example 3 of the present disclosure, where the Y-axis represents a cumulative release percentage (%), and the X-axis represents time (day);
Fig. 6 shows an in vitro cumulative release curve of a compound sustained-release preparation of naltrexone and risperidone prepared in Comparative Example 1 of the present disclosure, where the Y-axis represents a cumulative release percentage (%), and the X-axis represents time (day);
Fig. 7 shows an in vitro cumulative release curve of a compound sustained-release preparation of naltrexone and risperidone prepared in Comparative Example 2 of the present disclosure, where the Y-axis represents a cumulative release percentage (%), and the X-axis represents time (day);
Fig. 8 shows a in vivo drug-time curve of the compound sustained-release preparation of naltrexone and risperidone prepared in Example 1 of the present disclosure, where the Y-axis represents a plasma concentration percentage (%), and the X-axis represents time (day); and
Fig. 9 shows a comparison diagram of sensitization data of animal models according to examples of the present disclosure, where the Y-axis represents a travelled distance (cm), and the X-axis represents time (day).

### Detailed Description of the Embodiments

The present disclosure will be further described below with reference to the drawings.

### Example 1

### 1. Preparation of risperidone microspheres

Preparation of risperidone microspheres by a W₁/O/W₂ double emulsion-solvent evaporation method
(1) 0.6 g of sodium chloride was dissolved in 3 mL of purified water to form an inner water phase.
(2) 3.5 g of risperidone and 3.5 g of PLA (M_{w} 80,000 Da) were dissolved in dichloromethane to form an oil phase in which the concentration of risperidone was 150 mg/mL and the concentration of PLA was 150 mg/mL.
(3) The inner water phase was added to the oil phase, and the mixture was ultrasonically emulsified to form a primary emulsion.
(4) The primary emulsion was added dropwise to an aqueous solution (containing 0.5% polyvinyl alcohol and 5% sodium chloride) according to a ratio of 1: 40, the mixture was thoroughly stirred at 1,000 revolutions/minute until uniform, the organic solvent was evaporated by stirring at 800 revolutions/minute at 35°C for 24 h to obtain sustained-release microspheres, and the microspheres were washed, collected, and dried.

### 2. Preparation of naltrexone microspheres

Preparation of naltrexone microspheres by an O/W emulsion-solvent evaporation method
(1) 3.5 g of naltrexone and 3.5 g of PLA (M_{w} 80,000 Da) were dissolved in dichloromethane to form an oil phase in which the concentration of naltrexone was 150 mg/mL and the concentration of PLA was 150 mg/mL.
(2) The oil phase was added dropwise to an aqueous solution (containing 0.5% polyvinyl alcohol and 10% sucrose) according to a ratio of 1: 40, the mixture was thoroughly stirred at 1,000 revolutions/minute until uniform, the organic solvent was evaporated by stirring at 800 revolutions/minute at 35°C for 24 h to obtain sustained-release microspheres, and the microspheres were collected and dried.

### 3. Mixing and compression

10 parts of prepared naltrexone microspheres, 1 part of prepared risperidone microspheres, and 0.05% magnesium stearate were mixed and compressed into tablets.

### 4. The tablets were coated by spraying to obtain a compound sustained-release preparation of naltrexone and risperidone.

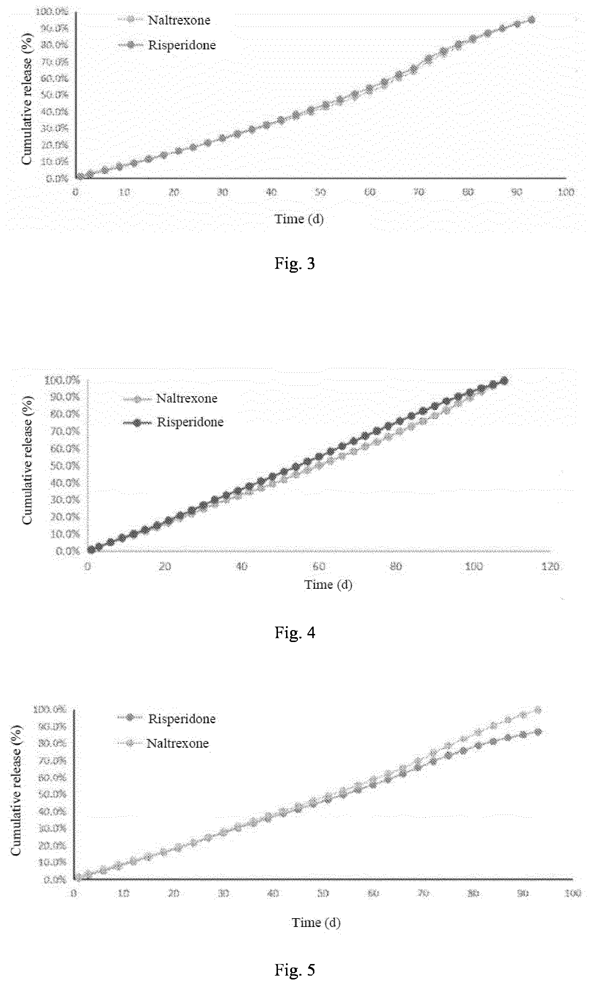

An in vitro cumulative release curve of the compound sustained-release preparation of naltrexone and risperidone prepared in this example is shown in Fig. 3, and it can be seen that the in vitro cumulative release period exceeds 90 days, and naltrexone and risperidone are released synchronously.

### Example 2

### 1. Preparation of risperidone microspheres

Preparation of risperidone microspheres by a W₁/O/W₂ double emulsion-solvent evaporation method
(1) 0.3 g of sodium dihydrogen phosphate was dissolved in 2 mL of water to form an inner water phase.
(2) 4.0 g of risperidone and 6.0 g of PLGA (lactic acid: glycolic acid = 90: 10, M_{w} 50,000 Da) were dissolved in dichloromethane to form an oil phase in which the concentration of risperidone was 100 mg/mL and the concentration of PLGA was 150 mg/mL.
(3) The inner water phase was added to the oil phase, and the mixture was ultrasonically emulsified to form a primary emulsion.
(4) The primary emulsion was added dropwise to an aqueous solution (containing 0.6% polyvinyl alcohol and 5% sodium chloride) according to a ratio of 3: 80, the mixture was thoroughly stirred at 700 revolutions/minute until uniform, the organic solvent was evaporated by stirring at 1,000 revolutions/minute at 35°C for 24 h to obtain sustained-release microspheres, and the microspheres were washed, collected, and dried.

### 2. Preparation of naltrexone microspheres

### Preparation of naltrexone microspheres by an O/W emulsion-solvent evaporation method

(1) 4.5 g of naltrexone and 6 g of PLGA (lactic acid: glycolic acid = 90: 10, M_{w} 50,000 Da) were dissolved in dichloromethane to form an oil phase in which the concentration of naltrexone was 150 mg/mL and the concentration of PLGA was 200 mg/mL.
(2) The oil phase was added dropwise to an aqueous solution (containing 1.0% polyvinyl alcohol and 8% sucrose) according to a ratio of 3: 80, the mixture was thoroughly stirred at 700 revolutions/minute until uniform, the organic solvent was evaporated by stirring at 1,000 revolutions/minute at 35°C for 24 h to obtain sustained-release microspheres, and the microspheres were collected and dried.

### 3. Mixing and compression

5 parts of prepared naltrexone microspheres, 1 part of prepared risperidone microspheres, and 0.1% sodium stearyl fumarate were mixed and compressed into tablets.

### 4. The tablets were coated by spraying to obtain a compound sustained-release preparation of naltrexone and risperidone.

An in vitro cumulative release curve of the compound sustained-release preparation of naltrexone and risperidone prepared in this example is shown in Fig. 4, and it can be seen that the in vitro cumulative release period exceeds 90 days, and naltrexone and risperidone are released synchronously.

### Example 3

### 1. Preparation of risperidone microspheres

Preparation of risperidone microspheres by a W₁/O/W₂ double emulsion-solvent evaporation method
(1) 0.5 g of disodium hydrogen phosphate was dissolved in 5 mL of water to form an inner water phase.
(2) 5.0 g of risperidone and 6.0 g of PLGA (lactic acid: glycolic acid = 50: 50, M_{w} 100,000 Da) were dissolved in ethyl acetate to form an oil phase in which the concentration of risperidone was 100 mg/mL and the concentration of PLGA was 120 mg/mL.
(3) The inner water phase was added to the oil phase, and the mixture was ultrasonically emulsified to form a primary emulsion.
(4) The primary emulsion was added dropwise to an aqueous solution (containing 0.5% polyvinyl alcohol and 15% sucrose) according to a ratio of 1: 40, the mixture was thoroughly stirred at 800 revolutions/minute until uniform, the organic solvent was evaporated by stirring at 700 revolutions/minute at 35°C for 24 h to obtain sustained-release microspheres, and the microspheres were washed, collected, and dried.

### 2. Preparation of naltrexone microspheres

### Preparation of naltrexone microspheres by an O/W emulsion-solvent evaporation method

(1) 5 g of naltrexone and 5 g of PLGA (50: 50, M_{w} 100,000 Da) were dissolved in dichloromethane to form an oil phase in which the concentration of naltrexone was 100 mg/mL and the concentration of PLGA was 100 mg/mL.
(2) The oil phase was added dropwise to an aqueous solution (containing 0.7% polyvinyl alcohol and 10% sucrose) according to a ratio of 1: 40, the mixture was thoroughly stirred at 900 revolutions/minute until uniform, the organic solvent was evaporated by stirring at 700 revolutions/minute at 35°C for 24 h to obtain sustained-release microspheres, and the microspheres were collected and dried.

### 3. Mixing and compression

5 parts of prepared naltrexone microspheres, 2 parts of prepared risperidone microspheres, 0.025% sodium stearyl fumarate, and 0.025% magnesium stearate were mixed and compressed into tablets.

4. The tablets were coated by spraying to obtain a compound sustained-release preparation of naltrexone and risperidone.

An in vitro cumulative release curve of the compound sustained-release preparation of naltrexone and risperidone prepared in this example is shown in Fig. 5, and it can be seen that the in vitro cumulative release period exceeds 90 days, and naltrexone and risperidone are released synchronously.

### Comparative Example 1 Preparation of risperidone microspheres by an O/W emulsion-solvent evaporation method different from Example 1

### 1. Preparation of risperidone microspheres

Preparation of risperidone microspheres by an O/W emulsion-solvent evaporation method
(1) 3 g of risperidone and 3 g of PLA (M_{w} 80,000 Da) were dissolved in dichloromethane to form an oil phase in which the concentration of risperidone was 150 mg/mL and the concentration of PLA was 150 mg/mL.
(2) The oil phase was added dropwise to an aqueous solution (containing 0.5% polyvinyl alcohol and 10% sucrose) according to a ratio of 1: 40, the mixture was thoroughly stirred at 1,000 revolutions/minute until uniform, the organic solvent was evaporated by stirring at 800 revolutions/minute at 35°C for 24 h to obtain sustained-release microspheres, and the microspheres were washed, collected, and dried.

### 2. Preparation of naltrexone microspheres

### Preparation of naltrexone microspheres by an O/W emulsion-solvent evaporation method

(1) 3 g of naltrexone and 3 g of PLA (M_{w} 80,000 Da) were dissolved in dichloromethane to form an oil phase in which the concentration of naltrexone was 150 mg/mL and the concentration of PLA was 150 mg/mL.
(2) The oil phase was added dropwise to an aqueous solution (containing 0.5% polyvinyl alcohol and 10% sucrose) according to a ratio of 1: 40, the mixture was thoroughly stirred at 1,000 revolutions/minute until uniform, the organic solvent was evaporated by stirring at 800 revolutions/minute at 35°C for 24 h to obtain sustained-release microspheres, and the microspheres were collected and dried.

### 3. Mixing and compression

10 parts of prepared naltrexone microspheres, 1 part of prepared risperidone microspheres, and 0.05% magnesium stearate were mixed and compressed into tablets.

4. The tablets were coated by spraying to obtain a compound sustained-release preparation of naltrexone and risperidone.

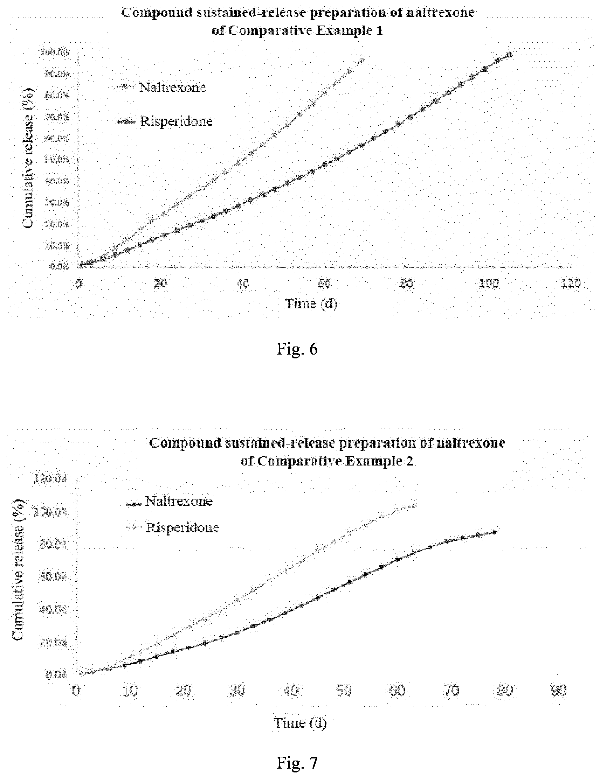

An in vitro cumulative release curve of the compound sustained-release preparation of naltrexone and risperidone prepared in this Comparative Example is shown in Fig. 6, and it can be seen that the in vitro cumulative release period is only 70 days, and naltrexone and risperidone cannot be released synchronously.

### Comparative Example 2 Preparation of naltrexone microspheres by a W₁/O/W₂ double emulsion-solvent evaporation method different from Example 1

### 1. Preparation of risperidone microspheres

Preparation of risperidone microspheres by a W₁/O/W₂ double emulsion-solvent evaporation method
(1) 0.6 g of sodium chloride was dissolved in 3 mL of purified water to form an inner water phase.
(2) 3.5 g of risperidone and 3.5 g of PLA (M_{w} 80,000Da) were dissolved in dichloromethane to form an oil phase in which the concentration of risperidone was 150 mg/mL and the concentration of PLA was 150 mg/mL.
(3) The inner water phase was added to the oil phase, and the mixture was ultrasonically emulsified to form a primary emulsion.
(4) The primary emulsion was added dropwise to an aqueous solution (containing 0.5% polyvinyl alcohol and 5% sodium chloride) according to a ratio of 1: 40, the mixture was thoroughly stirred at 1,000 revolutions/minute until uniform, the organic solvent was evaporated by stirring at 800 revolutions/minute at 35°C for 24 h to obtain sustained-release microspheres, and the microspheres were collected and dried.

### 2. Preparation of naltrexone microspheres

### Preparation of naltrexone microspheres by a W₁/O/W₂ double emulsion-solvent evaporation method

(1) 0.6 g of sodium chloride was dissolved in 4 mL of purified water to form an inner water phase.
(2) 3.5 g of naltrexone and 3.5 g of PLA (M_{w} 80,000 Da) were dissolved in dichloromethane to form an oil phase in which the concentration of naltrexone was 150 mg/mL and the concentration of PLA was 150 mg/mL.
(3) The inner water phase was added to the oil phase, and the mixture was ultrasonically emulsified to form a primary emulsion.
(4) The primary emulsion was added dropwise to an aqueous solution (containing 0.5% polyvinyl alcohol and 10% sucrose) according to a ratio of 1: 40, the mixture was thoroughly stirred at 1,000 revolutions/minute until uniform, the organic solvent was evaporated by stirring at 800 revolutions/minute at 35°C for 24 h to obtain sustained-release microspheres, and the microspheres were collected and dried.

### 3. Mixing and compression

10 parts of prepared naltrexone microspheres, 1 part of prepared risperidone microspheres, and 0.05% magnesium stearate were mixed and compressed into tablets.

4. The tablets were coated by spraying to obtain a compound sustained-release preparation of naltrexone and risperidone.

An in vitro cumulative release curve of the compound sustained-release preparation of naltrexone and risperidone prepared in this Comparative Example is shown in Fig. 7, and it can be seen that the release periods of naltrexone and risperidone are less than 80 days, and naltrexone and risperidone cannot be released synchronously.

It can be seen from Example 1 and Comparative Example 1 that in a case that the preparation methods of risperidone microspheres are different and the preparation methods of naltrexone microspheres are the same, the release behaviors of risperidone are similar, and the release behaviors of naltrexone are greatly different. Moreover, it can be seen from Example 1 and Comparative Example 2 that in a case that the preparation methods of risperidone microspheres are the same and the preparation methods of naltrexone microspheres are different, the release behaviors of naltrexone are similar, and the release behaviors of risperidone are greatly different. It shows the complexity of the release behaviors of the two sustained-release microspheres of the present disclosure when mixed, and to achieve the synchronous release of the two sustained-release microspheres, the parameters need to be limited within the ranges required by the present disclosure.

Comparative Example 3 Preparation by using a fat-soluble polymer with a molecular weight different from Example 1

### 1. Preparation of risperidone microspheres

Preparation of risperidone microspheres by a W₁/O/W₂ double emulsion-solvent evaporation method
(1) 0.6 g of sodium chloride was dissolved in 3 mL of purified water to form an inner water phase.
(2) 3.5 g of risperidone and 3.5 g of PLA (M_{w} 5,000 Da) were dissolved in dichloromethane to form an oil phase in which the concentration of risperidone was 150 mg/mL and the concentration of PLA was 150 mg/mL.
(3) The inner water phase was added to the oil phase, and the mixture was ultrasonically emulsified to form a primary emulsion.
(4) The primary emulsion was added dropwise to an aqueous solution (containing 0.5% polyvinyl alcohol and 5% sodium chloride) according to a ratio of 1: 40, the mixture was thoroughly stirred at 1,000 revolutions/minute until uniform, the organic solvent was evaporated by stirring at 800 revolutions/minute at 35°C for 24 h to obtain sustained-release microspheres, and the microspheres were collected and dried.

### 2. Preparation of naltrexone microspheres

### Preparation of naltrexone microspheres by an O/W emulsion-solvent evaporation method

(1) 3.5 g of naltrexone and 3.5 g of PLA (M_{w} 5,000 Da) were dissolved in dichloromethane to form an oil phase in which the concentration of naltrexone was 150 mg/mL and the concentration of PLA was 150 mg/mL.
(2) The oil phase was added dropwise to an aqueous solution (containing 0.5% polyvinyl alcohol and 10% sucrose) according to a ratio of 1: 40, the mixture was thoroughly stirred at 1,000 revolutions/minute until uniform, the organic solvent was evaporated by stirring at 800 revolutions/minute at 35°C for 24 h to obtain sustained-release microspheres, and the microspheres were collected and dried.

### 3. Mixing and compression

10 parts of prepared naltrexone microspheres, 1 part of prepared risperidone microspheres, and 0.05% magnesium stearate were mixed and compressed into tablets.

4. The tablets were coated by spraying to obtain a compound sustained-release preparation of naltrexone and risperidone.

In this case, naltrexone and risperidone cannot be released synchronously.

### Example In vitro release

An appropriate amount of naltrexone reference was taken and accurately weighed, a release medium was added to dissolve the naltrexone reference and quantitatively dilute to obtain a solution containing about 50 µg/mL naltrexone reference, and the solution was taken as a naltrexone reference solution.

An appropriate amount of risperidone reference was taken and accurately weighed, a release medium was added to dissolve the risperidone reference and quantitatively dilute to obtain a solution containing about 50 µg/mL risperidone control, and the solution was taken as a risperidone reference solution.

The product of the present disclosure was taken and placed into six 100 mL conical flasks with stoppers, 80 mL of 0.1 M anhydrous dipotassium hydrogen phosphate solution (a solution prepared by accurately sucking and placing 25 mL of 1 M sodium hydroxide solution into a 1,000 mL volumetric flask, adding a 0.1 M anhydrous dipotassium hydrogen phosphate solution for dilution, and regulating the pH value of the diluted solution to 11.2±0.05) was used as a solvent, and the conical flasks were tightly covered with the stoppers and shaken in a water bath oscillator at 100 times/minute at 37+0.5°C. The medium was replaced on day 1, day 3, day 6, day 9, day 12, day 15, ..., and day 120, the solution was filtered, a subsequent filtrate was taken as a test solution, and the release medium was replenished in the operating container in time. The test solution was detected by high-performance liquid chromatography (a high-performance liquid chromatography method described in General Rule 0512 of Chinese Pharmacopoeia 2020) at a wavelength of 280 nm.

In vitro cumulative release results are shown in Fig. 3 to Fig. 7, and it can be seen that the release period of the product of the present disclosure is more than 12 weeks, and the naltrexone and risperidone drugs are released synchronously. However, the two drugs cannot be released synchronously in Comparative Examples, and a difference between release end points is more than 2 weeks.

### Example Pharmacokinetics in animals

SD rats, half male and half female, were taken as experimental animals. The naltrexone compound preparation sample of Example 1 was used. The compound sustained-release preparation was implanted into the back of each rat, and blood of each rat was collected at set time points for analysis of drug-time data.

Blood collection method: the rat was anesthetized with isoflurane, about 1 mL of blood was collected from the retroorbital venous plexus, the collected whole blood was placed into an EP tube containing heparin immediately, the EP tube was shaken by reversing top and bottom three times, and placed in crushed ice, and centrifugation was performed (at 4,000 rpm at 4°C for about 5 min) within 1 h, and the centrifuged plasma sample was stored below -20°C. Naltrexone and its metabolite 6β-naltrexol, and risperidone and its metabolite 9-hydroxyrisperidone in the blood sample were detected by LC-MS/MS. Blood was collected for analysis once a week at time points 0h, 4h, D1, D3, D7, D10, D16, D39, D45, D60, D75, and D90.

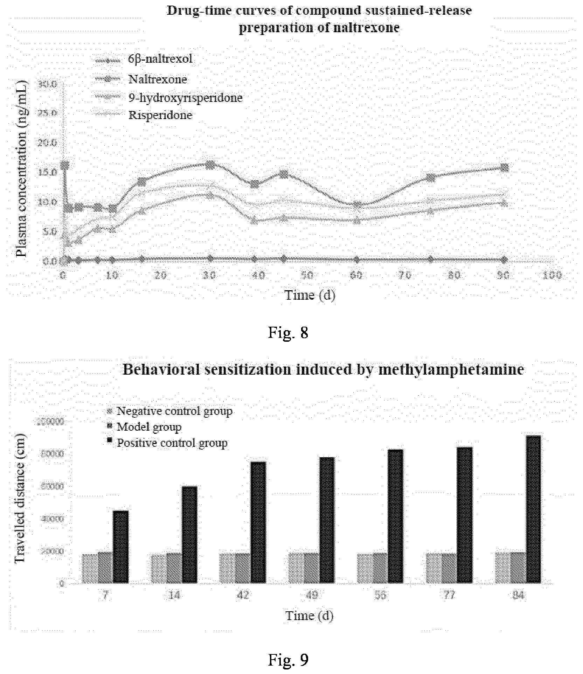

Drug-time analysis results are shown in Fig. 8, and it can be seen that the product of the present disclosure can effectively release risperidone and naltrexone into the blood in the body of the experimental animal, and the plasma concentration is kept stable within 90 days.

### Example Animal pharmacodynamic evaluation

All mice were bred under the same conditions. The product of the present disclosure was surgically implanted into each mouse in a model group before an experiment. Except implantation of the product of the present disclosure, other experimental conditions of a positive control group were the same as those of the model group. Mice in a negative control group were normally bred without undergoing experimental treatment. Behavioral differences among the model group, the positive control group, and the negative control group were observed during the experiment.

36 SD mice weighing 120-150 g, half male and half female, were taken. The mice were randomly divided into 3 groups: a positive control group, a negative control group, and a model group. The compound sustained-release preparation (prepared in Example 1) was implanted into the back of each mouse in the model group before administration, and a challenge experiment was carried out on the second day after surgery.

During the formative period, methylamphetamine (2 mg/kg) was daily intraperitoneally injected into each mouse in the positive control group and the model group, an equal amount of normal saline was intraperitoneally injected into each mouse in the negative control group for 7 consecutive days. During the conversion period, the drug was withdrawn for 7 days without any treatment. The expression period lasted for 12 weeks, during the expression period, methylamphetamine (2 mg/kg) was intraperitoneally injected into each mouse every 7 days for stimulation, and spontaneous activities of the mice were observed 30 minutes after injection.

Results are shown in Fig. 9, and it can be seen that from day 7, activities of the mice in the positive control group are significantly increased (P<0.05), which indicates that spontaneous activities of the mice chronically administered with methylamphetamine are obvious, and methylamphetamine can induce behavioral sensitization. Compared with the positive control group, spontaneous activities of the mice in the model group are significantly reduced (P<0.01) on day 7, day 14, day 42, day 49, day 56, day 77, and day 84, which are similar to those of the mice in the negative control group. Compared with the mice in the model group on day 7, spontaneous activities of the mice in the model group on day 14, day 42, day 49, day 56, day 77, and day 84 are not gradually increased as those of the mice in the positive control group, which indicates that after the pre-implantation of the compound sustained-release preparation of naltrexone and risperidone, the mice are antagonistic to methamphetamine, and the body is protected.

## Claims

1. A compound sustained-release composition of naltrexone and risperidone, comprising the following components in parts by weight:
5-10 parts of naltrexone sustained-release microspheres; and
1-2 parts of risperidone sustained-release microspheres,
the risperidone sustained-release microspheres being prepared by a W₁/O/W₂ double emulsion-solvent evaporation method, and the naltrexone sustained-release microspheres being prepared by an O/W emulsification-solvent evaporation method.

2. The composition according to claim 1, wherein preparation raw materials of the risperidone sustained-release microspheres comprise risperidone, an additive, a fat-soluble polymer, and a water-soluble polymer; preferably, the additive is selected from at least one of sodium chloride, mannitol, disodium hydrogen phosphate, and sodium dihydrogen phosphate; the fat-soluble polymer is selected from at least one of polylactic acid, poly(lactic-co-glycolic acid), polycaprolactone, polycarbonate, polyglycolic acid, polycyanoacrylate, and polyether ester; the water-soluble polymer is selected from polyvinyl alcohol; in the poly(lactic-co-glycolic acid), a ratio of lactic acid to glycolic acid is (1-9): 1; and the weight-average molecular weight of the water-soluble polymer or the fat-soluble polymer is 6,000-220,000 Da, and preferably 50,000-100,000 Da.

3. The composition according to claim 1, wherein a preparation method of the risperidone sustained-release microspheres comprises the following steps:
preparation of risperidone sustained-release microspheres by a W₁/O/W₂ double emulsion-solvent evaporation method, specifically comprising the following steps:
S1: dissolving an additive in purified water to form an inner water phase;
S2: dissolving risperidone and a fat-soluble polymer in an organic solvent to form an oil phase;
S3: adding the inner water phase to the oil phase, and performing ultrasonic emulsification to obtain a primary emulsion; and
S4: adding the primary emulsion to an aqueous solution of a water-soluble polymer, stirring until uniform, solidifying, evaporating the organic solvent, collecting, and drying to obtain risperidone sustained-release microspheres.

4. The composition according to claim 3, wherein in S1, a dosage ratio of the additive to the purified water is 50-300 mg/mL; in S2, the concentration of risperidone is 100-300 mg/mL, the organic solvent is selected from at least one of dichloromethane and ethyl acetate, and the concentration of the fat-soluble polymer is 100-300 mg/mL; and in S4, a dosage ratio of the primary emulsion to the aqueous solution of the water-soluble polymer is (1-3): 80, and in the aqueous solution of the water-soluble polymer, the content of the water-soluble polymer is 0.1-2 wt%, and preferably 0.5-0.6 wt%.

5. The composition according to claim 4, wherein in S4, the aqueous solution contains sodium chloride whose content is ≤5 wt% or sucrose whose content is ≤15 wt%, the stirring is performed at 500-5,000 revolutions/minute, and preferably 700-1,000 revolutions/minute, and the evaporation is performed at 200-3,000 revolutions/minute, preferably 500-1,000 revolutions/minute, at 30-40°C for 24 h.

6. The composition according to claim 1, wherein preparation raw materials of the naltrexone sustained-release microspheres comprise naltrexone, a fat-soluble polymer, and a water-soluble polymer; preferably, the fat-soluble polymer is selected from at least one of polylactic acid, poly(lactic-co-glycolic acid), polycaprolactone, polycarbonate, polyglycolic acid, polycyanoacrylate, and polyether ester; the water-soluble polymer is selected from polyvinyl alcohol; in the poly(lactic-co-glycolic acid), a ratio of lactic acid to glycolic acid is (1-9): 1; and the weight-average molecular weight of the water-soluble polymer or the fat-soluble polymer is 6,000-220,000 Da, and preferably 50,000-100,000 Da.

7. The composition according to claim 1, wherein a preparation method of the naltrexone sustained-release microspheres comprises the following steps:
preparation of naltrexone sustained-release microspheres by an O/W emulsion-solvent evaporation method, specifically comprising the following steps:
S1': dissolving naltrexone and a fat-soluble polymer in an organic solvent to form an oil phase; and
S2': adding dropwise the oil phase to an aqueous solution of a water-soluble polymer, stirring until uniform, solidifying, evaporating the organic solvent, collecting, and drying to obtain naltrexone sustained-release microspheres.

8. The composition according to claim 7, wherein in S1', the concentration of naltrexone is 50-300 mg/mL, the concentration of the fat-soluble polymer is 100-300 mg/mL, and the organic solvent is selected from at least one of dichloromethane and ethyl acetate; and in S2', a dosage ratio of the oil phase to the aqueous solution of the water-soluble polymer is (1-3): 80, in the aqueous solution of the water-soluble polymer, the content of the water-soluble polymer is 0.1-2 wt%, and preferably 0.5-1 wt%, the aqueous solution contains 0-10 wt% sodium chloride or 0-20 wt% sucrose, and preferably 8-10 wt% sucrose, the stirring is performed at 1,000-5,000 revolutions/minute, and preferably 700-1,000 revolutions/minute, and the evaporation is performed at 200-3,000 revolutions/minute, preferably 500-1,000 revolutions/minute, at 30-40°C for more than 24 h.

9. A preparation comprising the composition according to any one of claims 1 to 8 prepared by uniformly mixing the composition with a lubricant, compressing into tablets, and coating; preferably, the lubricant being selected from at least one of sodium stearyl fumarate, magnesium stearate, and a stearic acid, and a dosage of lubricant being 0.05-1 wt% of the composition.

10. The composition according to any one of claims 1 to 8 or the preparation according to claim 9 for use in treating or relieving amphetamine, ketamine, cocaine or cannabis addiction.

## Patentansprüche

1. Eine gemischte Zusammensetzung mit verzögerter Freisetzung von Naltrexon und Risperidon, die folgenden Komponenten in Gewichtsteilen umfassend:
5-10 Teile Naltrexon-Mikrosphären mit verzögerter Freisetzung; und
1-2 Teile Risperidon-Mikrosphären mit verzögerter Freisetzung,
wobei die Risperidon-Mikrosphären mit verzögerter Freisetzung durch ein W₁/O/W₂-Doppelemulsions-Lösungsmittel-Verdampfungsverfahren hergestellt werden und die Naltrexon-Mikrosphären mit verzögerter Freisetzung durch ein O/W-Emulgierungs-Lösungsmittel-Verdampfungsverfahren hergestellt werden.

2. Zusammensetzung nach Anspruch 1, wobei die Ausgangsmaterialien zur Herstellung der Risperidon-Mikrosphären mit verzögerter Freisetzung Risperidon, ein Additiv, ein fettlösliches Polymer und ein wasserlösliches Polymer umfassen; vorzugsweise ist das Additiv ausgewählt aus mindestens einer der Substanzen Natriumchlorid, Mannitol, Dinatriumhydrogenphosphat und Natriumdihydrogenphosphat; das fettlösliche Polymer ist ausgewählt aus mindestens einer der Substanzen Polymilchsäure, Poly(Milch-co-Glykolsäure), Polycaprolacton, Polycarbonat, Polyglykolsäure, Polycyanacrylat und Polyetherester; das wasserlösliche Polymer ist ausgewählt aus Polyvinylalkohol; in der Poly(Milch-co-Glykolsäure) beträgt ein Verhältnis von Milchsäure zu Glykolsäure (1-9):1; und das mittlere Molekulargewicht des wasserlöslichen Polymers oder des fettlöslichen Polymers beträgt 6000-220000 Da und vorzugsweise 50000-100000 Da.

3. Zusammensetzung nach Anspruch 1, wobei ein Herstellungsverfahren der Risperidon-Mikrosphären mit verzögerter Freisetzung die folgenden Schritte umfasst:
Herstellung von Risperidon-Mikrosphären mit verzögerter Freisetzung durch ein W₁/O/W₂-Doppelemulsions-Lösungsmittel-Verdampfungsverfahren, das insbesondere die folgenden Schritte umfasst:
S1: Auflösen eines Additivs in gereinigtem Wasser zur Bildung einer inneren Wasserphase;
S2: Auflösen von Risperidon und einem fettlöslichen Polymer in einem organischen Lösungsmittel zur Bildung einer Ölphase;
S3: Zugabe der inneren Wasserphase zur Ölphase und Durchführung einer Ultraschall-Emulgierung, um eine Primäremulsion zu erhalten; und
S4: Zugabe der Primäremulsion zu einer wässrigen Lösung eines wasserlöslichen Polymers, Rühren bis zur Einheitlichkeit, Verfestigung, Verdampfen des organischen Lösungsmittels, Sammeln und Trocknen, um Risperidon-Mikrosphären mit verzögerter Freisetzung zu erhalten.

4. Zusammensetzung nach Anspruch 3, wobei in S1 ein Dosierungsverhältnis des Additivs zu dem gereinigten Wasser 50-300 mg/ml beträgt; in S2 beträgt die Konzentration von Risperidon 100-300 mg/ml, das organische Lösungsmittel ist aus mindestens einem von Dichlormethan und Ethylacetat ausgewählt und die Konzentration des fettlöslichen Polymers beträgt 100-300 mg/ml; und in S4 beträgt ein Dosierungsverhältnis der Primäremulsion zu der wässrigen Lösung des wasserlöslichen Polymers (1-3):80, und in der wässrigen Lösung des wasserlöslichen Polymers beträgt der Gehalt des wasserlöslichen Polymers 0,1-2 Gew.-% und vorzugsweise 0,5-0,6 Gew.-%.

5. Zusammensetzung nach Anspruch 4, wobei in S4 die wässrige Lösung Natriumchlorid mit einem Gehalt von ≤5 Gew.-% oder Saccharose mit einem Gehalt von ≤15 Gew.-% enthält, das Rühren mit 500-5000 Umdrehungen/Minute, vorzugsweise 700-1000 Umdrehungen/Minute, durchgeführt wird und die Verdampfung bei 200-3000 Umdrehungen/Minute, vorzugsweise 500-1000 Umdrehungen/Minute, und bei 30-40 °C für 24 h durchgeführt wird.

6. Zusammensetzung nach Anspruch 1, wobei die Ausgangsmaterialien zur Herstellung der Naltrexon-Mikrosphären mit verzögerter Freisetzung Naltrexon, ein fettlösliches Polymer und ein wasserlösliches Polymer umfassen; vorzugsweise ist das fettlösliche Polymer ausgewählt aus mindestens einer der Substanzen Polymilchsäure, Poly(Milch-co-Glykolsäure), Polycaprolacton, Polycarbonat, Polyglykolsäure, Polycyanacrylat und Polyetherester; das wasserlösliche Polymer ist ausgewählt aus Polyvinylalkohol; in der Poly(Milch-co-Glykolsäure) beträgt ein Verhältnis von Milchsäure zu Glykolsäure (1-9):1; und das mittlere Molekulargewicht des wasserlöslichen Polymers oder des fettlöslichen Polymers beträgt 6000-220000 Da und vorzugsweise 50000-100000 Da.

7. Zusammensetzung nach Anspruch 1, wobei ein Herstellungsverfahren der Naltrexon-Mikrosphären mit verzögerter Freisetzung die folgenden Schritte umfasst:
Herstellung von Naltrexon-Mikrosphären mit verzögerter Freisetzung durch ein O/W-Emulsions-Lösungsmittel-Verdampfungsverfahren, das insbesondere die folgenden Schritte umfasst:
S1': Lösen von Naltrexon und einem fettlöslichen Polymer in einem organischen Lösungsmittel, um eine Ölphase zu bilden; und
S2': tropfenweise Zugabe der Ölphase zu einer wässrigen Lösung eines wasserlöslichen Polymers, Rühren bis zur Einheitlichkeit, Verfestigung, Verdampfen des organischen Lösungsmittels, Sammeln und Trocknen, um Naltrexon-Mikrosphären mit verzögerter Freisetzung zu erhalten.

8. Zusammensetzung nach Anspruch 7, wobei in S1' die Konzentration von Naltrexon 50-300 mg/ml beträgt, die Konzentration des fettlöslichen Polymers 100-300 mg/ml beträgt und das organische Lösungsmittel ausgewählt ist aus mindestens einem von Dichlormethan und Ethylacetat; und in S2' beträgt ein Dosierungsverhältnis der Ölphase zu der wässrigen Lösung des wasserlöslichen Polymers (1-3):80, in der wässrigen Lösung des wasserlöslichen Polymers beträgt der Gehalt des wasserlöslichen Polymers 0,1-2 Gew.-%, vorzugsweise 0,5-1 Gew.-%, die wässrige Lösung enthält 0-10 Gew.-% Natriumchlorid oder 0-20 Gew.-% Saccharose, vorzugsweise 8-10 Gew.-% Saccharose, das Rühren wird bei 1000-5000 Umdrehungen/Minute, vorzugsweise 700-1000 Umdrehungen/Minute durchgeführt, und die Verdampfung wird bei 200-3000 Umdrehungen/Minute, vorzugsweise 500-1000 Umdrehungen/Minute, und bei 30-40 °C für mehr als 24 h durchgeführt.

9. Eine Zubereitung, die die Zusammensetzung nach einem der Ansprüche 1 bis 8 umfasst, hergestellt durch gleichmäßiges Mischen der Zusammensetzung mit einem Gleitmittel, Komprimieren zu Tabletten und Beschichten; wobei das Gleitmittel vorzugsweise aus mindestens einem von Natriumstearylfumarat, Magnesiumstearat und einer Stearinsäure ausgewählt ist und die Dosierung des Gleitmittels 0,05-1 Gew.-% der Zusammensetzung beträgt.

10. Zusammensetzung nach einem der Ansprüche 1 bis 8 oder die Zubereitung nach Anspruch 9 zur Verwendung bei der Behandlung oder Linderung von Amphetamin-, Ketamin-, Kokain- oder Cannabisabhängigkeit.

## Revendications

1. Composition à libération prolongée de naltrexone et de rispéridone, comprenant les composants suivants en parties en poids :
5 à 10 parties de microsphères à libération prolongée de naltrexone ; et
1-2 parties de microsphères à libération prolongée de rispéridone,
les microsphères à libération prolongée de rispéridone étant préparées par un procédé d'évaporation de solvant en émulsion double W₁/O/W₂, et les microsphères à libération prolongée de naltrexone étant préparées par un procédé d'évaporation de solvant en émulsion H/E.

2. Composition selon la revendication 1, dans laquelle les matières premières de préparation des microsphères à libération prolongée de rispéridone comprennent la rispéridone, un additif, un polymère liposoluble et un polymère hydrosoluble ; de préférence, l'additif est choisi parmi au moins l'un parmi le chlorure de sodium, le mannitol, l'hydrogénophosphate disodique et le dihydrogénophosphate de sodium ; le polymère liposoluble est choisi parmi au moins l'un parmi l'acide polylactique, le poly(acide lactique-co-glycolique), la polycaprolactone, le polycarbonate, l'acide polyglycolique, le polycyanoacrylate et le polyétherester ; le polymère hydrosoluble est choisi parmi l'alcool polyvinylique ; dans le poly(acide lactique-co-glycolique), le rapport de l'acide lactique à l'acide glycolique est de (1-9) : 1 ; et le poids moléculaire moyen en poids du polymère hydrosoluble ou du polymère liposoluble est de 6 000 à 220 000 Da, et de préférence de 50 000 à 100 000 Da.

3. Composition selon la revendication 1, dans laquelle un procédé de préparation des microsphères à libération prolongée de rispéridone comprend les étapes suivantes :
préparation de microsphères à libération prolongée de rispéridone par un procédé d'évaporation de solvant en émulsion double W₁/O/W₂, comprenant spécifiquement les étapes suivantes :
S1 : dissolution d'un additif dans de l'eau purifiée pour former une phase aqueuse interne ;
S2 : la dissolution de la rispéridone et d'un polymère liposoluble dans un solvant organique pour former une phase huileuse ;
S3 : ajouter la phase aqueuse interne à la phase huileuse, et effectuer une émulsification par ultrasons pour obtenir une émulsion primaire ; et
S4 : ajouter l'émulsion primaire à une solution aqueuse d'un polymère hydrosoluble,
agiter jusqu'à uniformité, solidifier, évaporer le solvant organique, collecter et sécher pour obtenir des microsphères à libération prolongée de rispéridone.

4. Composition selon la revendication 3, dans laquelle dans S1, un rapport de dosage de l'additif à l'eau purifiée est de 50 à 300 mg/mL; dans S2, la concentration de rispéridone est de 100 à 300 mg/mL, le solvant organique est choisi parmi au moins l'un du dichlorométhane et de l'acétate d'éthyle, et la concentration du polymère liposoluble est de 100 à 300 mg/mL; et dans S4, un rapport de dosage de l'émulsion primaire à la solution aqueuse du polymère hydrosoluble est de (1-3) : 80, et dans la solution aqueuse du polymère hydrosoluble, la teneur en polymère hydrosoluble est de 0,1-2 % en poids, et de préférence de 0,5-0,6 % en poids.

5. Composition selon la revendication 4, dans laquelle dans S4, la solution aqueuse contient du chlorure de sodium dont la teneur est ≤5 % en poids ou du saccharose dont la teneur est ≤15 % en poids, l'agitation est effectuée à 500-5 000 tours/minute, et de préférence à 700-1 000 tours/minute, et l'évaporation est effectuée à 200-3 000 tours/minute, de préférence à 500-1 000 tours/minute, à 30-40 °C pendant 24 h.

6. Composition selon la revendication 1, dans laquelle les matières premières de préparation des microsphères à libération prolongée de naltrexone comprennent la naltrexone, un polymère liposoluble et un polymère hydrosoluble ; de préférence, le polymère liposoluble est choisi parmi au moins l'un parmi l'acide polylactique, l'acide poly(lactique-co-glycolique), la polycaprolactone, le polycarbonate, l'acide polyglycolique, le polycyanoacrylate et l'ester de polyéther ; le polymère hydrosoluble est choisi parmi l'alcool polyvinylique ; dans l'acide poly(lactique-co-glycolique), le rapport de l'acide lactique à l'acide glycolique est de (1-9) : 1 ; et le poids moléculaire moyen en poids du polymère hydrosoluble ou du polymère liposoluble est de 6 000 à 220 000 Da, et de préférence de 50 000 à 100 000 Da.

7. Composition selon la revendication 1, dans laquelle un procédé de préparation des microsphères à libération prolongée de naltrexone comprend les étapes suivantes :
préparation de microsphères à libération prolongée de naltrexone par un procédé d'évaporation de solvant en émulsion H/E, comprenant spécifiquement les étapes suivantes :
S1': la dissolution de la naltrexone et d'un polymère liposoluble dans un solvant organique pour former une phase huileuse ; et
S2': ajout goutte à goutte de la phase huileuse à une solution aqueuse d'un polymère hydrosoluble, agitation jusqu'à uniformité, solidification, évaporation du solvant organique, collecte et séchage pour obtenir des microsphères à libération prolongée de naltrexone.

8. Composition selon la revendication 7, dans laquelle, dans S1', la concentration de naltrexone est de 50 à 300 mg/ml, la concentration du polymère liposoluble est de 100 à 300 mg/ml, et le solvant organique est choisi parmi au moins l'un du dichlorométhane et de l'acétate d'éthyle ; et dans S2', un rapport de dosage de la phase huileuse à la solution aqueuse du polymère hydrosoluble est de (1-3) : 80, dans la solution aqueuse du polymère hydrosoluble, la teneur en polymère hydrosoluble est de 0,1-2 % en poids, et de préférence de 0,5-1 % en poids, la solution aqueuse contient 0-10 % en poids de chlorure de sodium ou 0-20 % en poids de saccharose, et de préférence 8-10 % en poids de saccharose, l'agitation est effectuée à 1 000-5 000 tours/minute, et de préférence 700-1 000 tours/minute, et l'évaporation est effectuée à 200-3 000 tours/minute, de préférence 500-1 000 tours/minute, à 30-40 °C pendant plus de 24 h.

9. Préparation comprenant la composition selon l'une quelconque des revendications 1 à 8, préparée en mélangeant uniformément la composition avec un lubrifiant, en la comprimant en comprimés et en l'enrobant ; de préférence, le lubrifiant étant choisi parmi au moins l'un parmi le stéaryl fumarate de sodium, le stéarate de magnésium et un acide stéarique, et une dose de lubrifiant étant de 0,05 à 1 % en poids de la composition.

10. Composition selon l'une quelconque des revendications 1 à 8 ou préparation selon la revendication 9 pour une utilisation dans le traitement ou le soulagement de la dépendance à l'amphétamine, à la kétamine, à la cocaïne ou au cannabis.
